Europäisches Patentamt

European Patent Office

Office européen des brevets

(11) Publication number: **0 132 309**
**A2**

(12) **EUROPEAN PATENT APPLICATION**

(21) Application number: **84304279.7**

(22) Date of filing: **25.06.84**

(51) Int. Cl.⁴: **C 12 N 15/00,** C 12 N 1/16

(30) Priority: **27.06.83 US 508426**

(43) Date of publication of application: **30.01.85**
**Bulletin 85/5**

(84) Designated Contracting States: **AT BE CH DE FR GB IT**
**LI LU NL SE**

(71) Applicant: **THE BOARD OF TRUSTEES OF THE LELAND**
**STANFORD JUNIOR UNIVERSITY, Office of Technology**
**and Licensing 105 Encina Hall Stanford University,**
**Stanford California 94305 (US)**

(72) Inventor: **Davis, Ronald W., 418 Concord Drive, Menlo**
**Park California 94305 (US)**
Inventor: **Johnston, Henry Mark, 954 Fremont Street,**
**Menlo Park California 94025 (US)**

(74) Representative: **Harrison, David Christopher et al,**
**MEWBURN ELLIS & CO 2/3 Cursitor Street, London**
**EC4A 1BQ (GB)**

(54) **Novel regulatable eukaryotic promoter element.**

(57) Novel DNA sequences useful as portable divergent promoters for inducible simultaneous transcription. Specifically, galactose-induced promoters for GAL1-10 genes are provided on a single fragment for use with foreign genes, where simultaneous transcription can be induced with galactose. Constructs are prepared providing self-replication in a eukaryotic host.

EP 0 132 309 A2

# NOVEL REGULATABLE EUKARYOTIC PROMOTER ELEMENT

There are ever increasing efforts to provide for expression of heterologous genes in a variety of hosts. A substantial amount of effort has been directed to providing replication systems which allow for replication of DNA in a multitude of either prokaryotic or eukaryotic hosts. Concomitant with the effort to produce vectors which will allow for the replication of genes foreign to a host have been efforts to provide promoters recognized by the host which allow for controlled regulation of efficient transcription of the foreign genes to provide enhanced production of the desired product. There has, therefore, been an ever expanding literature directed toward defining specific promoters and isolating such promoters to determine whether they can be used for competent regulation of a desired protein product.

St. John and Davis, Cell (1979) 16:443-452, describe the isolation of galactose-inducible DNA sequences from S. cerevisiae. St. John and Davis, J. Mol. Biol. (1981) 152:285-315 describe the organization and transcription of the galactose gene cluster of Saccharomyces. St. John et al., ibid. (1981) 152:317-334 describe the deletion analysis of the Saccharomyces GAL gene cluster and the transcription from three promoters.

In the invention externally inducible divergent promotors are provided as a portable DNA fragment for use in episomal elements for transcription of genes foreign to a host. Specifically, the GAL1-10 promotors are provided on a small DNS fragement which may be joined to the same or different foreign genes in opposite orientation on a _____

2                                          0132309

vector capable of stable maintenance in a host.  The
resulting construct can be used for the inducible
simultaneous transcription and expression of genes of
interest.

In accordance with the subject invention, DNA
fragments are provided having divergent bidirectional
promoters subject to simultaneous external regulation.
By having genes expressing the same or different
polypeptides downstream from the two promoters, concurrent transcription and expression of the proteins can
be obtained, so that higher amounts of a desired
product may be obtained or simultaneous production of
two different products is obtained, where the products
may be related by being subunits of the same protein,
by being related enzymes in a biosynthetic pathway or
by providing coordinate control of a plurality of other
regulatory systems.  Repression, derepression or
activation may be achieved through such external
effects as the use of co-repressors, inducers, or the
like.

The DNA fragment containing the divergent
promoters will generally be under 2kb, usually under
1.5kb and may be as small as 1kb or smaller.  Generally,
the promoters will be separated by at least about
100bp, more usually at least about 200bp and generally
from about 500-800bp.  Conveniently, the intervening
bases may serve as binding sites for regulatory proteins
which serve to regulate the transcription initiated by
the divergent promoters.  Thus, the fragment may serve
as a regulatable divergent promoter, which may be
joined to other sequences to provide for transcription
and expression of a variety of DNA sequences, frequently
structural genes.  The fragment may be modified in a
wide variety of ways to provide for various signals
associated with transcription and translation.

The presence of signals, such as capping sequences, signals involved with enhancing transcription, regulating transcription, and the like, the initiation site or codon, and portions of coding regions, such as leader sequences, processing signals, or the like, may also be present. In this way, one can provide for a cassette having a plurality of functions where simultaneous expression occurs.

As exemplary of the subject invention the yeast GAL1-10 promoters can be employed as a single inducible divergent promoter function, being joined to other DNA sequences to provide DNA constructs for the transcription and translation of DNA. The GAL1-10 promoters can be provided as a DNA fragment of less than about 1000bp and may be of any size greater than 1000bp. The intervening sequence between the GAL1-10 promoters generally will be less than about 650bp and more than about 200bp. The 1000bp dual promoter fragment, as obtained from the chromosome, provides for binding of RNA polymerase to provide for transcription, as well as sequences which provide for regulation of the transcription. This fragment by itself therefore provides for regulated divergent transcription of DNA sequences.

Regulation of transcription can be achieved using yeast hosts which provide repressors controlled by chemical or physical means. Various co-repressors for the gal genes include glucose, glycerol, and the product of the GAL80 gene. Positive regulation or activation can be achieved with inducers, such as galactose, or activators, such as the product of the GAL4 gene. Rather than using chemical regulation, one can employ temperature-sensitive mutations, which allow for regulation through repression and/or activation, by having temperature shifts, either up or down, generally in the range of from about 20°C to 50°C.

Extending downstream from the promoters may be a wide variety of flanking sequences, providing numerous functions. One can provide for cohesive ends or butt ends to the fragment, so as to be capable of ligation to other DNA sequences. By cleaving at various sites, one may retain various signals or coding sequences, depending upon particular situations. Cleavage will usually be upstream from the last base pair of the GAL1 and GAL10 genes. One can cleave immediately downstream from the promoter, so that one would join the divergent promoter function DNA sequence to other sequences which had the necessary transcriptional and translational signals.

Optionally, one can have a terminus at a site downstream from the promoter where all of the GAL transcriptional and translational regulatory signals downstream from the promoter have been removed. Alternatively, one can provide for retaining all of the GAL transcriptional and translational regulatory sequences downstream from the promoter, but remove the initiation codon and, optionally, a few bases upstream from the initiation codon, generally not more than about 20 bases, conveniently not more than 10 bases.

Finally, one may cleave in the GAL1 or GAL10 structural gene at a site which will permit joining of a DNA sequence in frame with the GAL1 and GAL10 initiation codons. In the last situation, one will obtain a fused protein, which may have advantages in many situations, particularly where there is some interest in having the GAL1 or GAL10 amino acid sequence at the N-terminus.

Of particular interest is the DNA fragment retaining the GAL1 and GAL10 transcriptional and translational regulatory sequences present between the promoter and the initiation codon and having different termini to simplify the joining of different DNA sequences at the two ends of the divergent promoter function fragment.

Therefore, by appropriate manipulation of the GAL1-10 genes and the intervening regulatory sequences, one can obtain a DNA fragment which provides for regulated transcription of foreign genes, by having a fragment which extends downstream from the promoter a major proportion of the DNA sequence intervening the initiation codons of the GAL1 and GAL10 genes, so that the transcriptional controls are endogenous to the yeast host. That is, the terminus of the fragment is upstream from the initiation codon but extends into the transcriptional leader sequence.

A number of vectors providing for intact replication systems are available for use in yeast. Many of the vectors are based on the replication system of the 2µ plasmid, by itself or in combination with ars1. A number of illustrative vectors may be found in Botstein et al., Gene (1979) 8:17-24. In addition to the yeast replication system, these vectors frequently have prokaryotic replication systems, which may be derived from episomal elements or phage. In this way, the manipulated or modified vector may be cloned in a prokaryotic host after each operation, and the desired construct isolated pure in relatively large amount.

Numerous capabilities may be introduced into the construct to provide for desirable traits. Desirably, markers are provided for each host in which the construct may be introduced, which markers may be the same or different. The markers normally provide for selection pressure, so as to ensure that the host retains the construct and does not become cured. Markers may include biocidal resistance, e.g. antibiotic resistance, colicin resistance, heavy metal resistance, etc., complementation of auxotrophy, immunity, etc. One or more markers may be desirable, depending upon the need for or the desirability of having different selective pressures.

A wide variety of foreign or heterologous genes may be employed to produce a large spectrum of different products. The heterologous genes may be derived from both prokaryotic and eukaryotic sources. Of particular interest are a wide variety of mammalian genes, from primates and domestic animals. Illustrative genes which are known and have been expressed in microorganism hosts include α-, β-, and γ-interferon, proinsulin and preproinsulin, human growth hormone, somatostatin, etc. Other genes of interest are those from pathogens, such as viruses and microorganisms, which expression products may be used for vaccines, for inducing antibody formation for use in passive immunization, or the like.

The following examples are offered by way of illustration and not by way of limitation.

<u>EXPERIMENTAL</u>

GAL DNA from <u>S</u>. <u>cerevisiae</u> was isolated and characterized as described in St. John and Davis, <u>J</u>. <u>Mol</u>. <u>Biol</u>. (1981) <u>152</u>:285-315. A yeast DNA fragment between the <u>EcoRI</u> site in <u>GAL10</u> and <u>AvaI</u> site in <u>GAL1</u>, which includes the divergent promoter for <u>GAL10</u> and <u>GAL1</u>, was isolated by gel electrophoresis. An <u>EcoRI</u> linker was joined to the <u>AvaI</u> site by T4 DNA ligase and the resulting fragment was cloned into YRp17. The resulting fragment was cleaved with <u>XbaI</u> and the linear molecule was digested for different lengths of time with the exonuclease Bal31. (Gray <u>et</u> <u>al</u>., <u>Nucleic</u> <u>Acids</u> <u>Res</u>. (1975) <u>2</u>:1459-1492; McKnight and Kingsbury, <u>Science</u> (1982) <u>217</u>:316-324.) <u>XbaI</u> synthetic linkers were ligated to the resulting molecules with T4 DNA ligase and recloned in a yeast vector. A random set of the resulting plasmid DNAs were first screened by restriction endonuclease cleavage to approximately identify the site of the deletion. Those near the start of transcription were then sequenced. Three deletions were identified that contained the synthetic XbaI linker at four bases before the authentic ATG for GAL1 and another clone was identified that contained the synthetic XbaI linker a few nucleotides upstream from the normal start of transcription. These two plasmids were then cleaved with XbaI.

The cohesive ends were filled in with DNA polymerase I and a BamHI linker added by T4 DNA ligase. The resulting molecule was then cleaved with BamHI and EcoRI and the fragment containing the divergent promoter was isolated by gel electrophoresis and inserted into the YCp50 vector. YCp50 vector was constructed from a parent plasmid called YIp5. See Struhl et al., PNAS USA (1979) 76:1035-1039. The EcoRI fragment containing the TRP1 gene and the ARS1 sequence was inserted into the unique EcoRI site of this vector. Also inserted was the EcoRI to BamHI fragment containing the centromere IV centrally located. The EcoRI site that joins the TRP1 ARS1 fragment to the CEN4-containing fragment was removed by partial digestion with EcoRI, filling in the cohesive ends with DNA polymerase I and covalently joining the blunt end molecules with T4 DNA ligase.

A fragment was then isolated which contains the ARS1 sequence fused to the CEN4 sequence by cleavage with HindIII followed by filling in the cohesive ends with DNA polymerase I and isolation of the fragment by gel electrophoresis. This fragment was then cleaved with PvuII and the fragment that contains the blunt end at the HindIII site in the TRP1 gene through ARS1 and CEN4 to the PvuII site was isolated by gel electrophoresis. This fragment was covalently joined to YIp5, which had been cleaved with PvuII, by T4 DNA ligase. The orientation of the fragment reads clockwise from 12 o'clock, TET resistance gene URA3, CEN4, ARS1, COL1 replicator, AMP resistance.

DNA from the two clones resulting from the two fragments inserted into the YCp50 vector were isolated and cleaved with EcoRI. The EcoRI site was filled in with DNA polymerase I and religated with T4 DNA ligase. This process removed the single EcoRI site and fused the divergent promoter to YCp50.

The direction for transcription for the GAL1 promoter was the clockwise at approximately 12 o'clock as described above.

Another clone was isolated by preparing a DNA fragment that comprised the DNA between the EcoRI site and GAL10 to the AvaI site in GAL1. The Ava site was converted to an EcoRI site by blunt end joining an EcoRI linker by T4 DNA ligase. The corresponding EcoRI fragment was inserted into YCp50. The resulting plasmid was partially digested with EcoRI, filled in with DNA polymerase I and rejoined and a molecule that had the EcoRI site between YCp50 and the EcoRI site in GAL10 missing was identified. The resulting molecule had a single unique EcoRI site in the GAL1 structural protein that was previously the AvaI site.

The above describes the preparation of three plasmid DNAs all constructed in YCp50 that have a single EcoRI site. One is a few bases upstream of the natural start of transcription for galactokinase or GAL1. The second contains the normal leader for GAL1, but stops four bases from the authentic ATG used for the galactokinase initiation. The third is in the structural gene for galactokinase.

Employing similar techniques, one can manipulate the downstream sequences of the GAL10 promoter, providing for different unique restriction sites, so that two different structural genes may be introduced at the two sites and controlled expression of the two genes achieved with simultaneous expression.

In accordance with the subject invention, novel constructs are provided where a promoter cluster is manipulated, so as to be useful for transcriptional regulation of genes. The divergent GAL1-10 promoters allow for simultaneously regulated transcription of two different genes or the same gene, whereby a wide variety of products may be produced in yeasts or other hosts which recognize the promoters.

Although the foregoing invention has been described in some detail by way of illustration and example for purposes of clarity of understanding, it will be obvious that certain changes and modifications may be practiced.

WHAT IS CLAIMED IS:

1. A DNA fragment of less than about 2000bp having approximately symmetrically located the yeast GAL1-10 divergent promoters.

2. A DNA fragment according to Claim 1, having termini at base pairs upstream from the terminal base pairs of the GAL1 and GAL10 structural genes.

3. A DNA fragment according to Claim 2, wherein said termini are upstream from the initiation codons.

4. A DNA fragment according to Claim 3, wherein said termini are within about 20 base pairs of said initiation codons.

5. A DNA construct comprising a DNA fragment having the yeast GAL1-10 divergent promoters and, in opposite orientations downstream from said promoters, genes foreign to yeast.

6. A DNA construct according to Claim 5, having a yeast replication system.

7. A DNA construct according to Claim 5, having a marker recognized by yeast.

8. A DNA construct according to Claim 5, having a yeast replication system, a prokaryotic replication system and at least one marker.

9. A yeast host having an episomal element containing a construct according to Claim 5.